(19) Europäisches Patentamt
European Patent Office
Office européen des brevets

(11) EP 0 381 742 B1

(12) EUROPEAN PATENT SPECIFICATION

(45) Date of publication and mention of the grant of the patent: **19.06.1996 Bulletin 1996/25**

(21) Application number: **89909342.1**

(22) Date of filing: **03.08.1989**

(51) Int. Cl.$^6$: **A61B 6/00**, A61K 49/00, G01N 24/00

(86) International application number: **PCT/US89/03352**

(87) International publication number: **WO 90/01295 (22.02.1990 Gazette 1990/05)**

# (54) RECEPTOR MEDIATED ENDOCYTOSIS TYPE MRI CONTRAST AGENTS

VON EINEM REZEPTOR INDUZIERTES KONTRASTMITTEL FÜR DEN ENDOCYTOSE-TYP MRI

AGENTS DE CONTRASTE D'IRM DE TYPE ENDOCYTOSE INDUITS PAR RECEPTEUR

(84) Designated Contracting States:
**AT BE CH DE FR GB IT LI LU NL SE**

(30) Priority: **04.08.1988 US 228640**
**28.07.1989 US 384991**

(43) Date of publication of application: **16.08.1990 Bulletin 1990/33**

(60) Divisional application: **95102752.3**

(73) Proprietor: **ADVANCED MAGNETICS, INC.
Cambridge, MA 02138-1038 (US)**

(72) Inventors:
- **MENZ, Edward, T.
Quincy, MA 02170 (US)**
- **ROTHENBERG, Jeffrey, M.
Gary, IN 46403 (US)**
- **GROMAN, Ernest, V.
Brookline, MA 02146 (US)**
- **JOSEPHSON, Lee
Arlington, MA 02174 (US)**

(74) Representative: **Warcoin, Jacques et al
Cabinet Régimbeau,
26, avenue Kléber
F-75116 Paris (FR)**

(56) References cited:

| | |
|---|---|
| EP-A- 0 169 299 | EP-A- 0 186 947 |
| EP-A- 0 236 619 | EP-A- 0 273 452 |
| WO-A-88/00060 | US-A- 4 647 447 |
| US-A- 4 675 173 | US-A- 4 822 594 |
| US-A- 4 849 210 | |

- **J. BIOCHEM., vol. 100, 1986, pages 1481-1492; S.B. SATO et al.: "A novel method for isolating specific endocytic vesicles using very fine ferrite particles coated with biological ligands and the high-gradient magnetic separation technique"**
- **HEPATOLOGY, vol. 8, no. 5, September-October 1988, page 1253, abstract no. 140, Baltimore, US; G. WU et al.: "A hepatocyte-directed contrast agent for magnetic resonance imaging of hepatic tumors"**
- **J. CANCER RES. CLIN. ONCOL., vol. 113, 1987, pages 51-55, Springer-Verlag, Berlin, DE; J. BEUTH et al.: "Inhibition of liver metastasis in mice by blocking hepatocyte lectins with arabinogalactan infusions and D-galactose"**
- **IRI REPORT, no. 133-75-07, pages 1-12; P.P. VAN RIJK et al.: "Preparation of iodine-131-labelled asialo. Alpha. 1-acid glycoprotein"**
- **FILE SERVER STN, Karlsruhe, FILE CHEMICAL ABSTRACTS, vol. 109, no. 25, abstract no. 225919a, Columbus, Ohio, US; G. GALLI et al.: "A radiopharmaceutical for the study of the liver: technetium-99m-DTPA-asialo-orosomucoid. II: Human dynamic and imaging studies"**
- **Physiological Chemistry and Physics and Medical NMR, Vol. 16, 1984, G. E. WESBEY et al., "Paramagnetic Pharmaceuticals for Magnetic Resonance Imaging", pp. 145-155. See page 146, column 2 to page 155, column 1.**
- **American Journal of Radiology, Vol. 148, February 1987, WIDDER et al., "Magnetite Albumin Microspheres: A New MR Contrast Material", pp. 399-404. See page 399, page 401, column 2 to page 403, column 2.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.12.4/3.4

- The New England Journal of Medicine, Vol. 297, No. 25, 22 December 1977, JACOBS et al., "Current Concepts", pp. 1383-1386. See page 1383, column 2 to page 1384, column 2.

## Description

### 1. FIELD OF THE INVENTION

The present invention relates to the synthesis and use of materials in magnetic resonance imaging. In particular, the compositions of the present invention serve as contrast agents for the enhancement of magnetic resonance (MR) images. The present invention describes a class of MR contrast agents which are directed to specific cells of the body based on the ability of these cells to perform receptor mediated endocytosis. MR agents whose biodistribution is based on receptor mediated endocytosis can enhance the general level of anatomical detail obtained with MR images both between different tissues or within a single tissue. In addition, because receptor mediated endocytosis is a metabolically regulated process, the extent of contrast agent uptake can provide information on the metabolic function of tissue volumes visualized with the MR imaging technique. In particular, the preparation of biodegradable superparamagnetic receptor mediated endocytosis type MR contrast agents is described. The in vivo utility of these contrast agents in MR techniques is demonstrated.

### 2. BACKGROUND OF THE INVENTION

#### 2.1. MAGNETIC RESONANCE IMAGING

In magnetic resonance imaging (MRI) an image of an organ or tissue is obtained by placing a subject in a strong magnetic field and observing the interactions between the magnetic spins of the protons and radiofrequency electromagnetic radiation. (For a review of MR imaging technique see Balter, S. RadioGraphics **1987**, 7(2) 371-383; Fullerton, G.D. RadioGraphics **1987**, 7(3), 579-596). Two parameters termed proton relaxation times are of primary importance in the generation of the image. They are called $T_1$ (also called the spin-lattice or longitudinal relaxation time) and $T_2$ (the spin-spin or transverse relaxation time). $T_1$ and $T_2$ depend on the chemical and physical environment of protons in various organs or tissues.

The utility of MR imaging techniques in the characterization and differentiation of pathologic from healthy tissues is most easily demonstrated in cases where divergent relaxation times occur within a region of interest. For example in cerebral tissue the protons of the cerebral spinal fluid have far different relaxation times from neural tissue and the resulting MR images are of high contrast.

In other instances the image produced may lack definition and clarity due to a similarity of the signal from different tissues or different compartments within a tissue. In some cases, the magnitude of these differences is small, limiting the diagnostic effectiveness of MR imaging. Thus, there exists a real need for methods which increase or magnify these differences. One approach to improving image quality is through the use of contrast agents.

#### 2.2. THE USE OF MR CONTRAST AGENTS

The field of MR image enhancement and the use of contrast agents are discussed extensively in applicants' co-pending Patent Application No. 067,586 which is a continuation-in-part of Applicants' Patent Application No. 882,044. The teachings and publications cited in these applications are incorporated in the instant application by reference. Presently, MR imaging contrast agents fall broadly under three categories: paramagnetic, ferromagnetic, and hyper- or superparamagnetic. Although a wide array of these substances has been investigated for their ability to serve as MR contrast agents, only a small fraction of the material so far reported will prove to have the clinical efficacy and margin of safety required for use in humans.

European Patent Application No. 0 186 616, for example, discloses a whole host of double metal oxide/hydroxide particles for use in diagnostic techniques, including "nmr-diagnosis." Many combinations of metals, of both divalent and trivalent oxidation states, are used along with a wide range of " physiological compatible complex former[s]", including polysaccharides, proteins, carboxylic acids, synthetic polymers, and even zeolites. This European Application is related to the published German patent Application DE 3443251A1, whose disclosures are much more limited. However, the processes and materials disclosed in these preceding foreign applications would not be expected to demonstrate any tissue specificity, and in particular no specificity for hepatocytes.

The earliest MR contrast agents developed include paramagnetic chelates which can alter both $T_1$ and $T_2$ and can be used to visualize the vascular compartment. The most studied compound of this class is a gadolinium chelate, Gd-DTPA, which has proven useful in the imaging of the brain by virtue of its ability to delineate regions where the blood brain barrier has broken down (See, Runge et al., Mag. Res. Imag. **1985**, 3, 43-55; U.S. Patent No. 4,647,447). A wide range of paramagnetic iron chelates are also listed in European Patent Application No. 0 186 947, PCT Application WO 85/0554, PCT Application WO 86/06605, European Patent Application No. 0 210 043, and U.S. Patent Application Nos. 4,639,365 and 4,637,929. These paramagnetic metal chelates are used primarily as intravascular MR contrast agents and have limited utility in imaging organs and tissues of the reticuloendothelial system (RES).

For visualizing parts of the reticuloendothelial system, MR contrast agents based on iron oxides have been developed. These RES-type MR contrast agents are not particularly tissue-specific because they are picked up by the phagocytic cells of the RES present primarily in the liver, spleen, lymphoid system, and the bone marrow. A variety of the magnetic particles can be used because the function of the RES is to remove dead cells, bacteria, and other particulate material from circulation (see U.S. Patent No. 4,675,173, PCT Applications WO 85/04330 and WO 85/02772, and European Patent Application 0 184 899). Most of these RES-type MR contrast agents employ ferromagnetic materials, but the superparamagnetic materials described by the applicants in their above-referenced co-pending U.S. Applications are highly preferred.

Still other types of MR contrast agents include immunodirectable materials. This work was spurred by the ability of radiolabeled antibodies to serve as in vivo diagnostic agents (see Renshaw, P.F. et al. Mag. Res. **1986**, 4, 351-357). Because some antigens are found only on specific types of cells, immunodirectable MR contrast agents might appear to be an attractive approach to the development of tissue-specific MR contrast agents. There are limitations to antibody-directed MR contrast agents, however. In particular only a limited number of fixed cell surface antigens exist in a specific tissue. As a result only a small proportion, typically only a few percent, of the immunodirectable MR contrast agent administered to the subject become bound to the target cells. A comprehensive review of approaches to MR contrast agent development has recently been compiled (Lauffer, R.B. Chem. Rev. **1987**, 87, 901-927).

2.3. LIGAND BINDING AND INTERNALIZATION IN CELLS

The mechanistic pathway for macromolecule recognition, binding, and internalization into the intracellular compartment is a subject of intense research in cell biology. Most review articles describing endocytosis and related processes for the uptake of extracellular material also discuss the structures of the vacuolar apparatus which take part in the internalization (See, for example, Steinman, R.M. et al., J. Cell Biol. **1983**, 96, 1-27; Wileman, T. et al., Biochem. J. **1985**, 232 1-14; Helenius, A. et al., Trends Biochem. Sci. **1983**, 8 245-249; Pastan, I.H. and Willingham, M.C., Ibid. **1983**, 8, 250-254).

Investigators in the field agree that the assimilation of physiologically significant molecules such as nutrients, hormones, enzymes, virions, toxins, and various types of proteins begins with the initial binding of the macromolecule or ligand to specific receptors which are mobile and randomly distributed on the cell membrane surface. These ligand-receptor complexes rapidly accumulate in specialized regions of the membrane termed coated pits. From this stage, the receptor-mediated endocytosis (RME) proceeds to the formation of smooth-walled vesicles which allow entry of the concentrated ligand-receptor complexes into the cell. These vesicles, often referred to as "endosomes" or "receptosomes," may fuse together or combine with larger vesicles. Subsequently, the internal pH of these endosomes decrease by the action of proton pumps, changing the conformation of the receptor and/or ligand. The result is the release of the ligand and the formation of separate receptor-containing vesicles and ligand-containing ones. In some cases, the receptor-bearing vesicles are delivered to the cell membrane where they are released and "recycled" for additional use. In others, the resulting vesicles, along with the internalized ligand, are delivered to and fused with lysosomes where the eventual breakdown likely takes place.

A feature of RME is that it is subject to regulation which reflects the metabolism of the cell. The ability of cells to upregulate (increase RME) or downregulate (decrease RME) is a sensitive indicator of their function. For a discussion of the importance of RME regulation in medicine, see Jacobs and Cuatrecasas, New Engl. J. Med. **1977**, 297, 1383 and Nature **1976**, 259, 265. A wide variety of molecules are internalized by RME, and for many of these substrates the requisite receptor is found in selected cells or in selected tissues. Thus, for the study of fibroblastic tissue, LDL, EGF, or mannose 6-phosphate glycoproteins are useful (See, for example, Pastan, I.H. and Willingham, M.C., Science **1981**, 214, 504-509; Anderson, R.G.W. et al., Cell **1977**, 10, 351-364; Murray, G.I. and Neville, Jr., D.M., J. Biol. Chem. **1980**, 255, 11942-11948; Sando, G.N. and Karson, E.M., Biochem. **1980**, 19, 3850-3855). In addition, a receptor for LDL mediates removal of cholesterol from plasma. Alterations in LDL receptor activity may be correlated with elevated serum cholesterol and with the development of atherosclerosis (See, Goldstein and Brown, Ann. Rev. Biochem. **1977**, 46, 897).

Transferrin receptors are located in a number of cell types but particularly in maturing erythroblasts and reticulocytes of the bone marrow (See, Ward, J.H., Invest. Radiol. **1987**, 22, 74-83; Harding, E. et al., J. Cell Biol. **1983**, 97, 329-339). Rapidly dividing cells (e.g. tumor cells) have increased transferrin receptor activity and sequester $^{67}$Ga after the radioactive material has bound transferrin. (Larson, S.M. in "Radiopharmaceuticals - Structure Activity Relationships," Spencer, R.P. ed, (Grune and Stratton, 1981), pp. 167-181)).

Removal of a terminal sialic acid from glycoproteins often exposes a galactose. This terminal galactose of the carbohydrate chain is recognized by a receptor on hepatocytes (Lee, Y.C. and Lee, R.T. in "The Glycoconjugates," Vol. 4, pp. 57-83, M.I. Horowitz, ed. (New York, 1982)). The asialoglycoprotein receptor withdraws a variety of molecules with terminal galactose from circulation and internalizes them within vacuoles of hepatocytes. This receptor characteristically disappears when hepatocytes are transformed to hepatoma cell (Schwartz, et al., J. Biol. Chem. **1981**, 256, 8878-8881) or in rapidly dividing or regenerating hepatocytes (Stockert, R.J. and Morell, A.G. in "The Liver: Biology and Pathobiology", pp. 205-217, I. Arias, H. Popper, D. Schacter and D.A. Shafritz eds. (New York, 1982)). The asialoglycoprotein receptor is therefore an excellent example of RME whose function reflects cell metabolism. Of course, the insulin receptor, responsible for regulating the diverse activities of the hormone is of great importance in the pathogenesis of obesity and diabetes (See, Gambhir et al., Clin. Chem. **1977**, 23, 1590).

Very recently, research carried out by Beuth, J. et al., Cancer Res. Clin. Oncol. **1987**, 113, 51-55, showed that liver metastasis in mice can be inhibited by blocking hepatocyte lectins with infusions of arabinogalactan or D-galactose. Surprisingly, other galactans were found by these workers to be ineffective liver metastatis inhibitors.

Interestingly, larger protein-protein conjugates, metal-neoglycoalbumin adducts, ternary, or higher order compositions like ferrite-BSA-asialofetuin (AsF) are all effectively bound by the surface receptors of their corresponding cell types. Thus, LDL-ferritin conjugates are useful in studies comparing normal fibroblasts with cells from a familial hypercholesterolemia homozygote (Anderson, R.G.W. et al., Proc. Natl. Acad. Sci. USA **1976**, 73, 2434-2438). In vivo animal studies have shown the high liver specificity of a radioisotope preparation containing $^{99m}$Tc-neoglycoalbumin (Vera, D.R. et al., J. Nucl. Med. **1985**, 26, 1157-1167). Asialoorosomucoid-gold and transferrin-gold complexes have been studied (Neutra, M.R. et al., J. Hist. and Cyto. **1985**, 33(11), 1134). Also, conjugates comprised of LDL adsorbed onto colloidal gold are stable probes for fibroblast receptors (Handley, D.A. et al., Proc. Natl. Acad. Sci. USA **1981**, 78, 368-371). And magnetic preparations using commercial ferrite coated with BAS-AsF are alleged to be useful in the magnetic isolation of murine hepatic endosomes (Sato, S.B. et al., Studia Biophysica **1985**, 110, 123-126; J. Biochem. **1986**, 100, 1481-1492). Finally, EPA 0,273,452 asserts that asialoglycoprotein acceptor-directing compounds can be conjugated to a chelate which is linked to radioactive metallic element in order to target the asialoglycoprotein receptor.

None of the complexes above have been directed to the preparation of biodegradable superparamagnetic RME-type MR contrast agents. In particular, labeled arabinogalactan species have not been described. Such tissue directable materials could serve as the basis for a diagnostic technique which provides valuable anatomical information, delineating inter alia the extent of injury or recovery of a given organ or tissue.

## 3. SUMMARY OF THE INVENTION

The present invention provides a new class of MR contrast agents and method which uses these contrast agents to enhance the quality of MR images. The contrast agents of the invention are capable of being distributed in vivo to selected organs or tissues of the subject by a particular cell recognition and internalization pathway.

Specifically, this invention is directed to colloidal biodegradable superparamagnetic metal oxide particles associated with a polysaccharide, which polysaccharide is capable of being recognized and internalized by an organ or tissue by receptor mediated endocytosis (RME), as contemplated in claims 1-15, and the use of the same as contrast agents in MR imaging, as contemplated in claim 16. Based on the ability of the polysaccharide to participate in RME, the contrast agents are, likewise, capable of participating in RME.

In one embodiment of the invention, a polysaccharide containing a terminal galactose moiety is labeled with the metal oxide particles. The resulting contrast agent, when administered parenterally, can exhibit a marked selectivity for the hepatocytes of the liver at the expense of other organs and tissues of the reticuloendothelial system (RES).

Because the uptake of these contrast agents is governed by RME, a different biodistribution is obtained compared with existing intravascular or RES-type contrast agents. The use of these RME-type contrast agents according to the methods of the invention affords superior anatomical detail both between different organs and tissues and within different compartments of the same organ or tissue.

It is also an object of the present invention to provide a diagnostic method, based on MR imaging, which affords valuable information drawn directly from the metabolic, physiological, or pathological condition of the organ or tissue under examination.

The invention further contemplates MR contrast agents directable to specific cells by recognition and internalization mechanisms other than RME.

## 4. BRIEF DESCRIPTION OF THE FIGURES

Figure 1. shows a series of MR images of a rat taken at different times after administration of the RME-type MR contrast agent of the invention.

Figure 2. shows the chromatogram obtained from a Sepharose 4B separation of the HS MR contrast agent (barbed line) and AMI-25 (solid-line).

Figure 3. shows the effects of administering RES-type (left) vs. HS-type (right) MR contrast agents on the blood relaxation rates of rats over time.

Figure 4. shows the effect of co-injected glycoproteins on the clearance of the HS MR contrast agent from the blood of rats : -+-, no co-injected glycoproteins ; -●-, co-injected with fetuin ; -■-, co-injected with asialofetuin.

FIG. 5 shows MR image of a rat before administration of contrast agent (upper left), after administration of 20 μmol/kg HS MR contrast agent (upper right), and after co-administration of 20 μmol/kg/HS and 100 μmol/kg AMI-25 MR contrast agents (lower left).

## 5. DETAILED DESCRIPTION OF THE INVENTION

In common with other types of MR contrast agents, RME-type MR contrast agents can enhance MR images by improving overall contrast (i.e., anatomical detail) both between tissues or within a given tissue. With the RME-type contrast agents, however, such an image may be obtained free from interference from nearby organs or surrounding fluid and employing an optimized dose versus toxicity characteristic of the MR contrast agent. Moreover, the localization and uptake of the RME-type contrast agent, and hence the resulting image, and governed by variations in the rate of RME which is, in turn, a function of the metabolic state or general health of the cell. Therefore, RME-type contrast agents can provide direct information regarding the functional state of the organ or tissue under examination, thereby improving the utility of the MR technique overall.

### 5.1 PREPARATION OF RECEPTOR-RECOGNIZED LIGANDS

The ligands useful in the instant invention are comprised of polysaccharides and may be comprised of synthetic but physiologically tolerable materials. Preferably, these polysaccharide-based ligands comprise natural polysaccharide biomolecules or slightly modified derivatives thereof. Suitable polysaccharide-based ligands may, therefore, be di-saccharides or oligosaccharides, such as β-D-galactopyranosyl-D-galactose, or α-D-galactosyl-α-D-galactosyl-α-D-glucosyl-β-D-fructose. Other polysaccharide-based ligands can be prepared by conjugating or modifying a macromolecular species, such as a polysaccharide, a carbohydrate or a polymerizable organosilane, with the appropriate polysaccharide to form a macromolecular species conjugate. Suitable organosilanes include aminopropyltrimethoxysilane, p-aminophenyltrimethoxysilane, N-2-aminoethyl-3-aminopropyltrimethoxysilane, n-dodecyltriethoxysilane, and n -hexyltrimethoxysilane.

In a preferred embodiment of the invention, an RME-type MR contrast agent designed to engage the asialoglycoprotein receptor (ASGPR) of hepatocytes is prepared using natural polysaccharide biomolecules or polysaccharide-containing conjugates with terminal galactose groups. These galactose groups can, therefore be contained in polysaccharides, functionalized dextran or organosilane conjugates.

In this fashion a number of specific tissues and organs can be selectively targeted. These "designer" contrast agents should find particular utility in the diagnosis of primary and secondary (metastatic) cancers, especially of the liver, in acute cases of cirrhosis and hepatitis, and in the prognosis of transplanted or traumatized livers. This invention seeks to provide an effective means for monitoring the progress of a given treatment and for examining organ and tissue damage arising from, for example, hereditary abnormalities, malnutrition, exposure to parasitic organisms, infections, harmful drugs, the presence of neoplastic diseases, chronic cirrhosis and hepatitis, or the simple degenerative breakdown of physiological mechanisms within the body.

It is also intended that the compositions used and the methods of the instant invention provide a direct measure of liver function and regional differences in the state of the liver.

Moreover, by the appropriate choice of ligand, the tissue-specific MR contrast agents may be designed to reside on the surface of the cell membrane for variable lengths of time, from seconds to hours.

Some substrates may be purchased from commercial sources. Hexoses, such as D- or L-galactose, -glucose, or -mannose are available, as are galactosamine, glucosamine, mannosamine hydrochlorides, and D-mannopyranosyl 6-phosphate. The p-aminophenyl derivatives of most hexoses are available also. Likewise, certain oligosaccharides, for example, lactose, maltopolyoses, and α-D-galactosyl-α-D-galactosyl-α-D-glucosyl-β-D-fructose, may be purchased.

A highly preferred macromolecular species useful in targeting the hepatocytes of the liver is the carbohydrate, arabinogalactan. This galactose-containing carbohydrate is available from commercial sources and may be derivatized, like other carbohydrates, by methods well-known in the art.

With the proper choice of ligand, RME-type contrast agents can be tailored to reside on the cell surface receptor for variable lengths of time before the onset of endocytosis.

Other starting materials may be prepared by adapting methods well known in the art. Some references are provided in the Examples Section of this application, infra.

### 5.2. PREPARATION OF SUPERPARAMAGNETICALLY-LABELED RME-TYPE MR CONTRAST AGENTS

The superparamagnetic label is comprised of aggregates of individual biodegradable superparamagnetic metal oxide crystals and is prepared preferably in the hydrated state from a combination of trivalent and divalent metal cationic salts. These superparamagnetic crystals are typically 5-10 nm in diameter, but upon clustering, the resulting aggregates may be as large as 1000 nm. It is important to note that the larger the overall dimension of the aggregate, the higher is the likelihood that these larger particles are removed from the extracellular fluid by phagocytosis. This condition is generally avoided by employing relatively small aggregates, those having volume median diameters (as measured by light scattering) 100 nm or less, preferably 50 nm or less. At these dimensions, the biodistribution of the RME-type contrast agent based upon the recognition and internalization by cells having a particular ligand-sensitive surface receptor is fully exploited.

Most advantageously, the biodegradable superparamagnetic metal oxide is made from iron, but other metals including cobalt, chromium, molybdenum, manganese, nickel, vanadium, tungsten, or copper, to name a few, may be selected. The receptor-recognized polysaccharide or macromolecular species is associated to the metal oxide aggregates by a procedure which preferably precipitates the metal oxides in the presence of the desired ligand species. Hence solutions containing the appropriate amounts of trivalent and divalent metal salts and the selected polysaccharide-based ligands (e.g. oligo- or polysaccharides; or carbohydrate or organosilane macromolecular species conjugates) are combined and treated with aqueous ammonium hydroxide solution.

The dark superparamagnetic composition which forms is collected by centrifugation, resuspended, and subjected to sonication. Excess or unreacted reagents are generally removed by dialysis. The physiologically acceptable carriers or media which can be used in the invention are those generally recognized and known in the art. These carriers may include, but are not limited to, aqueous solutions, saline, buffered solutions, solutions containing polycarboxylic acid salts and the like, or mixtures thereof. The size distribution of the associated particles may be determined by light scattering methods, X-ray diffraction, electron microscopy or other suitable analytical method.

It has further been discovered that heating the freshly precipitated metal oxide composition to a temperture of about 90°C to about 100°C provides metal oxide crystal aggregates having smaller volume median diameters as determined by light scattering, while also having a somewhat narrower overall size distribution. This modified procedure, as described further in Section 6.3, provides higher yields of material based on iron and need not be subjected to a sonication step. These "colloidal" metal oxide preparations, like their earlier counterparts, have a maximum overall mean diameter (or volume median diamemter) of about 5000 angstroms (500 nm). Preferably, these aggregates fall below 3000 angstroms (300 nm), and most preferably, below 1000 angstroms (1000 nm). Of the available macromolecular species, arabinogalactan has been found to give the best results with respect to targeting the hepatocytes of the liver (more, infra).

Also, and as already mentioned previously, it may be desirable to replace at least part of the divalent iron in a colloidal preparation of biodegradable superparamagnetic iron oxide with other divalent metals, preferably zinc, manganese, or cobalt. Typically, when synthesizing contrast agents in which the metal is iron and in which the ligand contains a galactose-terminal moiety, the galactose/iron molar ratio should not be less than about 0.0001 and not greater than about 1.0.

### 5.3 DETERMINATION OF THE IN VIVO DISTRIBUTION OF RME-TYPE MR CONTRAST AGENTS AND THEIR ENHANCEMENT OF MR IMAGES

The superparamagnetically-labeled materials of this invention can be prepared with radioisotopes such as $^{14}C$, $^{123}I$, or $^{125}I$. After the MR agents are administered to test animals, tissue samples can be obtained and the level of radioactivity is measured. Different levels of radioactivity for different tissue samples indicate the relative specificity of the RME-type MR contrast agent for the respective tissues or organs.

A more satisfactory approach involves the imaging of the mouse or patient, and the direct determination of proton relaxation times using a magnetic resonance spectrometer. The instrument may be focused on individual organs and tissues and their $T_1$ and/or $T_2$ values are measured readily. A comparison of these values provides a gauge for the specificity of the RME-Type MR contrast agents. The results of these MR experiments provide useful information in and of themselves. If so desired the actual MR image may then be generated as part of the MR experiment.

An image of the organ or tissue under examination can be generated and manipulated with the aid of numerous pulse sequence and data-acquisition techniques. The final result can be dependent on the type of tissue being investigated and the disease state. In the case of hepatocyte directed contrast agents, the number of functioning ASGPRs can be a measure of the condition of the liver. It is regarded, for example, that a characteristic loss of ASGPRs and the corresponding loss of ability to endocytose asialoglycoproteins is associated, generally, with transformed hepatocytes (i.e., hepatoma cells). Less magnetic material is expected to concentrate in the transformed tissue and, therefore, these areas are anticipated to be lighter in shade than the surrounding healthy tissue.

By contrast, cells that utilize a large amount of iron (e.g., developing cells or rapidly-dividing ones) possess a greater number of transferrin receptors. Thus, it may be expected that images of tumorous growths in the region of the bone marrow may appear darker than surrounding untransformed tissue. In this manner, information useful in diagnosing the metabolic state of an organ or tissue may be obtained.

It has been found that a MR contrast agent which is exceptionally selective for the hepatocytes of the liver can be obtained from a colloidal superparamagnetic metal oxide associated with a macromolecular species comprising arabinogalactan, a galactose-containing carbohydrate (See, for example, Beuth, J. et al. in Cancer Res Clin Oncol **1987**, 113, 51-55). Administration of this "hepatocyte-specific" MR (HS MR) contrast agent in mice in amounts effective to cause a marked change in the relaxation times of hepatic tissue protons has a negligible effect on the relaxation times of splenic tissue (See, Table IV). This specificity has been observed despite the propensity of colloidal superparamagnetic iron oxides to be taken up by the spleen as found for the RES-type MR contrast agents.

The rate of clearance of this HS MR contrast agent from the blood is sensitive to the presence of circulating asialo- (or galactose-terminal)-glycoproteins. As evident from FIG. 4, pre- or co-injection of asialofetuin inhibited the clearance of the contrast agent while the use of fetuin, a non-galactose-terminal glycoprotein, had no effect on the rate of clearance. From this sensitivity to circulating galactose-terminal moieties (D-galactose is also expected to affect the clearance rate of the contrast agent) and the observed liver hepatocyte specificity, one may conclude, justifiably, that the contrast agent is being selectively removed from the blood by the asialoglycoprotein receptor of hepatocytes.

It should be pointed out, however, that the interpretation of the blood clearance of a given compound, in terms of hepatic receptor activity, is complicated by the possibility that a slow clearance rate from the blood may reflect not a decreased hepatic receptor activity but a decreased hepatic blood flow. This latter situation arises when a particular compound has a high first pass extraction efficiency; that is, a compound is removed substantially after a single pass through the hepatic capillary bed, with very little of the compound reaching the hepatic receptor sites. Conversely, a low first pass extraction efficiency allows hepatic metabolic activity to be the rate-limiting step in the clearance of a compound from the blood, rather than the organ (liver) blood flow.

Although the first pass extraction efficiency of the present HS MR contrast agent is hard to determine, especially with a blood half-life of only 4.9 minutes, there is ample reason to believe that organ blood flow will not be a significant factor in determining the metabolic state of the organ as a function of the contrast agent uptake. First, the dosage used in MR imaging is generally high enough to guarantee that a significant proportion of the administered contrast agent will reach the target receptors. If not, the dosage can be increased particularly because the present preparations are of such low toxicity. Alternatively, receptors in the capillary beds may be competitively bound by pre- or co-administration of receptor-blocking agent. As mentioned previously, such blocking agents may include, but are not limited to galactose, neoglycoalbumin, and other galactose-terminal moieties such as asialofetuin or arabinogalactan itself.

A dose of as little as 20 µmol Fe/kg of the subject rat has been found effective to provide an enhaced MR imgage. This dosage corresponds to 78 mg Fe for a 70 kg man. Furthermore, the toxicity studies undertaken have shown that the $LD_{50}$ of the HS MR contrast agent is at least 1800 µmol/kg rat, providing a safety factor of at least 90. These "coated" superparamagnetic colloids, like their RES-type predecessors, have the metal comprising about 50% of the colloids' mass, with the remainder constituting oxygen and the associated macromolecule. Thus, about 160 mg of the HS MR contrast agent is needed to produce an enhanced image. This dose is markedly less than that (gram quantities) which would be required to effect a similar result using asialoglycoproteins labeled with a paramagnetic metal (See, for example, Wu, G.Y. et al. in Hepatology **1988**, 8, 1253).

Moreover, further improvements on synthetic technique, MR pulse-sequence methods, and a reduction in the lag time between administration of the contrast agent and actual acquisition of data, may reduce the dosage requirements still further. The pulse sequence employed to record the image presented in FIG. 5, may be described as a mid $T_1$-$T_2$ weighted spin-echo pulse sequence. Highly $T_2$ weighted spin-echo, or gradient echo pulse sequences, have also been used to record the effect of hepatic superparamagnetic iron oxide.

The following examples are meant to illustrate further embodiments of the present invention and should not be construed as limiting its scope or utility in any manner.

## 6. EXAMPLES

### 6.1. SYNTHESIS OF SUPERPARMAGNETIC RME-TYPE MR CONTRAST AGENTS REFERENCE EXAMPLE:

#### 6.1.1 SYNTHESIS OF SUPERPARAMAGNETIC ASIALOFETUIN

Into a clean centrifuge tube containing 20 mL of distilled water is added 1.25 mL each of an aqueous solution of asialofetuin (1 mg/mL) and an aqueous solution of bovine serum albumin (1 mg/mL). To the resulting solution is added, simultaneously, 175 µL of an aqueous solution containing 35 mg of ferrous chloride tetrahydrate and 140 µL of an aqueous solution containing 70 mg of ferric chloride hexahydrate. The resulting mixture is vortexed and then made alkaline (pH ~8.5) by the addition of a sufficient amount of aqueous 7.5% ammonium hydroxide solution (ca. 500 µL). The black precipitate which forms is collected by centrifugation (3000 rpm x 10 min), and the supernatant is discarded. The pellet is washed by adding aqueous $KH_2PO_4$ (pH 7.0, 40 mL), vortexing, and centrifuging the suspension. The washing procedure is repeated a total of three times.

After the third and final wash, the pellet is resuspended once more with the neutral potassium phosphate buffer, transferred to a beaker, and sonicated for about 90-120 sec using a Branson 184 V sonicator probe. Light scattering measurements indicate that the volume median diameter (VMD) of the particles decreases from about 800-1000 nm to about 100 nm (1000 angstroms) during the period of sonication. The technique also indicates that the particles tend to be non-uniform in size. Other techniques for determining the size distribution may also be employed such as electron microscopy. Analysis of the dry weight of the particle indicates the presence of about 5% by weight protein.

6.2. REFERENCE EXAMPLE BIODISTRIBUTION OF BIODEGRADABLE SUPERPARAMAGNETIC RME-TYPE MR CONTRAST AGENTS

The biodistribution of RME-type contrast agents may be determined by injecting them into vertebrate animals, sacrificing the animals after a short period of time, and determining the effect on the proton relaxation times of the tissues of interest. This general method can be illustrated for the case of MR contrast agents recognized and selectively internalized via galactose receptors of hepatocytes of the liver.

A Sprague Dawley rat is injected through the tail vein with an RME-type contrast agent (AsF/BSA) prepared according to Section 6.1.1 (2 mg Fe/kg of rat). After about 2 h, the animal is sacrificed and the liver and spleen are removed. Tissue samples from control animals and those injected with a superparamagnetic iron oxide (AMI-25, RES-type), or injected with a BSA-associated superparamagnetic iron oxide (RES-type), are obtained likewise. The $T_2$ proton relaxation times of the various tissues are determined using an IBM PC-20 pulsed MR spectrometer. The results are listed in Table I.

TABLE I

Proton Relaxation Times of Tissues Removed from Rats Used in Biodistribution Experiments[a]

| Contrast Agent | $T_2$ (msec) | | $1/T_2$ ($sec^{-1}$) | |
|---|---|---|---|---|
| | Liver | Spleen | Liver | Spleen |
| Normal | 36.5 | 65 | 27.4 | 15.4 |
| AsF/BSA[b] | 24 | 52 | 42.7 | 19.2 |
| AMI-25[c] | 11.7 | 16 | 85.5 | 62.5 |
| Normal | 32 | 48 | 31.3 | 20.8 |
| BSA[d] | 20 | 22 | 50.0 | 45.4 |

[a]Data from two sets of experiments are shown. [b]RME-type contrast agent prepared according to Example 6.1.1. of the invention. [c]Citrated superparamagnetic fluid prepared according to U.S. Application Serial No. 067,586. [d]BSA superparamagnetic iron oxide complex prepared according to U.S. Patent No. 4,770,183.

The results indicate that while the RES-type contrast agents (AMI-25 and BSA particle) dramatically affect the proton relaxation times of tissues derived from both the liver and spleen of treated rats, the RME-type contrast agent exhibits a marked preference for the liver tissue. Only slight changes in the proton relaxation rates are observed in the spleen of normal rats versus those administered with the RME-type contrast agent. These results demonstrate that the RME-type contrast agent directed specifically to galactose receptor-bearing cells are taken up selectively in vivo by hepatocytes, which hepatocytes are found only in the liver tissue of animals and humans.

A distribution coefficient, D, can be defined by the expression written below (Eq. 1):

$$D = \frac{1/T_2\ \text{(treated liver)} - 1/T_2\ \text{(normal liver)}}{1/T_2\ \text{(treated spleen)} - 1/T_2\ \text{(normal spleen)}} \qquad \text{(Eq.1)}$$

in which $1/T_2$ is the relaxation rate obtained from the results of Table I. The magnitude of the term D provides an indication of the selectivity of a given RME-type MR agent for a given pair to tissue (i.e., the liver versus the spleen in the case supra). The larger the number the more selectively the agent is distributed in favor of one tissue. Table II lists the values of D calculated from the above-mentioned experiments, along with other pertinent information.

Table II

Distribution Coefficient, D, Calculated for Different Kings of MR Contrast Agents

| Contrast Agent | D | Dose (mg Fe/kg rat) | Time[a] (h) | VMD[b] (nm) |
|---|---|---|---|---|
| AsF/BSA | 4.0 | 2 | 2 | 101 |
| AMI-25 | 1.23 | 2 | 1 | 70 |
| BSA | 0.76 | 2 | 1.5 | 100 |

[a]Time elapsed after administration of contrast agent.

[b]Volume median diameter obtained from light scattering method.

It is apparent from the data in Table II that the RES-type contrast agents (last two entries) do not manifest the type of selectivity enjoyed by the RME-type (first entry) agent. The AsF/BSA particle is about 3 times more selective for the liver relative to AMI-25 and about 5 times more selective compared with a particle associated with BSA only. When employing hepatocyte specific contrast agents which comprise particles measuring 3000 angstroms (300 nm) or less and a ligand containing a terminal galactose moiety, the contrast agents of the instant invention can display D values of at least about 3 and even D values of at least about 20.

6.3. HEPATOCYTE SPECIFIC (HS) MR CONTRAST AGENTS

6.3.1. PREPARATION OF HEPATOCYTE-SPECIFIC COLLOIDAL SUPERPARAMAGNETIC METAL OXIDE MR CONTRAST AGENTS

An aqueous solution of trivalent and divalent metal salts is prepared as exemplified by the use of the following amounts of ferric and ferrous halide salts: $FeCl_3$ 6 $H_2O$ (15.8 g, 58.5 mmol) and $FeCl_2$ $4H_2O$ (6.24 g, 31.6 mmol) are combined in distilled water (200 mL) and the resulting solution is then filtered through a 0.22 μm glass fiber filter to remove large debris. Equal volumes of this metal halide solution and a carbohydrate solution, prepared by dissolving arabinogalactan from larch wood (60 g, Sigma Chemical Co.) in distilled water (120 mL), are then combined at ambient temperature with vigorous stirring. To this mixture is then added, slowly and dropwise, a 30% aqueous ammonium hydroxide solution until the pH of the mixture reaches about 10. At this stage, the mixture is heated to a temperature about 90°-100°C for about 15 minutes. The mixture is then allowed to cool with the formatiom of a black colloidal superparamagnetic iron oxide. The cooled mixture is then passed through a coarse glass fiber filter, followed by a series of Nalgene® filters of decreasing porosity beginning with an 0.8 μm, then an 0.45 μm, and finally an 0.22 μm filter.

Excess arabinogalactan is then removed by ultrafiltration using a 2 liter hollow fiber dialysis unit having a 300 kilodalton molecular weight cutoff (Amicon Inc., Danvers, MA) as follows: the filtered product from the preceding step is loaded into the ultrafiltration unit, diluted and washed with 25 mM citrate buffer (pH 8.5). This washing step is repeated until a clear eluent is observed (about 5 cycles). The washed product is then concentrated to a final volume which is about equal to the initial volume of the combined metal salt and carbohydrate solutions. The final product, in which the yield based on iron is about 90%, is preferably refrigerated until needed.

6.3.2. PHYSICAL CHARACTERIZATION OF THE HEPATOCYTE-SPECIFIC MR CONTRAST AGENT

The size distribution of the final product, obtained by the procedure described in the preceding Section, may be determined in one of several ways. For example, and as mentioned previusly in Section 6.1.1, light-scattering techniques offer a useful way of estimating the size distribution of a given sample. Electron microscopy (EM) is perhaps the preferred means for a reliable determination of the sizes and size distribution of suspended particles or colloids. It has been the experience of the present inventors that the volume median diameter (VMD, sometimes also referred to herein as the overall mean diameter), obtained from the light scattering experiments, is most closely correlated to the EM values.

More conveniently, however, gel filtration chromatography, using, for example, Sepharose 4B as the stationary phase, provides more than adequate quantitative determinations when the resulting fractions are correlated with samples of known size. In the present case, the final product is analyzed on a 100 x 0.3 cm Sepharose column. For comparative purposes, a sample of superparamagnetic metal oxide (AMI-25), prepared according to the methods described in U.S. Patent 4,827,945, is analyzed similarly. The results are plotted in FIG. 2 (the solid line is the AMI-25 and the barbed line represents the size distribution of the HS MR contrast agent). The largest superparamagnetic particles are present in the early fractions, as exemplified by the excluded volume (fraction 28), and have been found, by light scattering, to have a VMD of about 120 nm. Ferritin, on the other hand, elutes with a peak at fraction 59 (not shown) close to the smallest of the superparamagnetic particles. This ferritin sample is known to have a diameter of about 10-15 nm by electron microscopy. One may conclude, therefore, that both the superparamagnetic particles prepared according to the previous methods, and the HS MR contrast agents of the present embodiments, have a size distribution between about 10 and about 120 nm. However, it is clear from FIG. 2 that the majority of HS MR contrast agent clusters are confined to a somewhat narrower and smaller size distribution relative to the superparamagnetic iron oxide (also referred to as "citrated") material. This difference in size and size distribution may be attributed to the additional heating step described in Section 6.3.1.

These two materials are subjected to further analyses including VMD determinations by light scattering methods (using an instrument, Model BI-90, available from Brookhaven Instruments, Inc., Ronkonkoma, NY), their effects on proton relaxation times (using a PC-20 MR Spectrometer operating at 0.47 Tesla and available from IBM Instruments, Danbury, CT), and magnetic susceptibility as determined using a susceptibility balance (Johnson Matthey Inc., West Chester, PA). The results of these additional analyses are listed in Table III.

TABLE III

| Physical characteristics of a Colloidal Superparamagnetic HS MR Contrast Agent and a Citrated Superparamagnetic Reticuloendothelial System (RES)-Type MR Contrast Agent | | |
|---|---|---|
| | MR Contrast Agent | |
| | HS | RES[d] |
| Size[a] (nm) | 54 | 72 |
| $R_1$[b]($M^{-1}$ $sec^{-1}$) | 2.1 x $10^4$ | 3 x $10^4$ |
| $R_2$[b]($M^{-1}$ $sec^{-1}$) | 5.2 x $10^4$ | 10 x $10^4$ |
| Magnetic Susceptibility[c] (c.g.s.) | 15.5 x $10^{-3}$ | 25.0 x $10^{-3}$ |
| Surface Species | galactose | citrated |

[a]The size refers to the volume median diameter (overall mean diameter) as determined from light scattering methods of unfractionated material. [b]$R_1$ and $R_2$ are the spin-lattice and spin-spin relaxivities which reflect the molar change in $1/T_1$ or $1/T_2$, produced by the MR contrast agents, respectively. [c]The magnetic susceptibility is the weight susceptibility per gram of iron. [d]The RES-type MR contrast agent (AMI-25) is prepared according to the methods described in U.S. Patent No. 4,827,945.

### 6.4. IN VIVO RESULTS: BIODISTRIBUTION AND PHARMACOKINETICS OF THE HS MR CONTRAST AGENTS

#### 6.4.1. METHODS

One of two methods are used for administering the HS MR Contrast Agents and for anesthesizing the Sprague-Dawley rats (350 g, available from Charles River Labs, Wilmington, MA) used in these in vivo experiments. First, the rats are anesthesized with an intraperitoneal injection of sodium pentabarbital (35 mg/kg dose). A lateral incision is made, and the HS MR contrast agent is injected into the vena cava. Blood samples are extracted from that vein. The data shown in FIG. 3 and Table IV reflect the results obtained from this first method.

In the second method, a long acting anesthetic, Inactin (100 mg/kg dose), is injected intraperitoneally. The femoral artery and vein of the rats are then exposed by a small incision. The artery is then carefully separated from the vein. The HS MR Contrast Agent is subsequently injected into the femoral vein. For the blood lifetime studies, the artery is cannulated and 0.5 mL blood samples are then collected periodically.

The relaxation times are recorded at 38° ± 1°C on the PC-20 MR instrument at 0.47 Tesla. $T_1$ is measured from eight data points generated with an inversion recovery pulse sequence. $T_2$ is measured from 10 data points with a Carr-Purcell-Meiboom-Gill pulse sequence (See, for example, Weissleder, R. W. et al. Amer. J. Roentgen. **1989**, 152, 167-173; $T_1$ and $T_2$ are measured in this instrument as described therein).

MR images are obtained with a 2 Tesla GE CSI 45 cm bore unit with a time of repetition (TR) setting of 500 msec and a time to echo (TE) setting of 30 msec.

Also, the metal concentration of a given sample may also be measured, if desired, on an atomic absorption spectrophotometer, such as one manufactured by Perkin-Elmer Corp., Norwalk, CT), after dissolution of the metal oxide in 0.01 M HCl.

#### 6.4.2. RESULTS

FIG. 3 shows the changes in blood spin-lattice relaxation rate ($1/T_1$) following the injection of 40 µmol/kg of either HS MR contrast agent or citrated RES-type MR contrast agent. The introduction of superparamagnetic colloids into blood produces a change in the blood relaxation rate which is proportional to the concentration of added superparamagnetic colloid over a wide concentration range; i.e., the change in $1/T_1$ is proportional to the blood concentration of the superparamagnetic colloid. All points in FIG. 3 are averaged over 6 rats. The blood half life is then calculated by fitting the data to a single exponential equation: $1/T_{1(t)} = 1/T_{1(base)} + Ae^{-kt}$. Here $1/T_{1(base)}$ and $1/T_{1(t)}$ are the spin-lattice relaxation rates of blood before injection of the superparamagnetic colloid or at some time (t) after injection. A is the concentration of the contrast agent in the blood at the moment of injection, and k is the rate constant for the decay of $1/T_1$ in the blood following injection. The blood half-life is then calculated from the value of k.

Employing the data shown in FIG. 3 and carrying the calculations through, the blood-half-life for the HS MR contrast agent is 4.9 minutes (95% confidence limits). For the RES-type agent, a blood half-life of 6.4 minutes is obtained (95% confidence limits). Thus, both contrast agents are rapidly cleared from the blood. Blood half-lives based on measurements of $1/T_2$, are calculated in a similar fashion and are found to be 3.9 min and 6.7 min for the HS and RES-type MR contrast agents, respectively.

A hepatocyte directable or hepatocyte specific MR contrast agent should ideally be cleared from blood only by the liver, the source of hepatocytes, while other superparamagnetic RES-type MR contrast agents, like AMI-25, are cleared from the blood by the phagocytic action of the RES (e.g., Kupffer cells of the liver). In rats, radiotracer studies indicate that the highest concentration of AMI-25 is found in the spleen (See, for example, Weissleder, R.W. et al. Amer. J. Roentgen. **1989**, 152, 167-173).

The hepatic and splenic relaxation rates ($1/T_1$'s and $1/T_2$'s) resulting from the injection of 20 μmol/kg of either the HS MR agent or AMI-25 are shown in Table IV. All animals are sacrificed 60 minutes after injection of the contrast agent. The HS MR agent caused large changes in the relaxation rates of the liver, without causing a significant change in the splenic values of $1/T_1$ and $1/T_2$. As expected, the RES-Type MR agent, AMI-25, produced large changes in the relaxation rates of both the liver and the spleen.

The ratio of hepatic uptake to splenic uptake provides a simple measure of the degree of hepatocyte specificity of the HS agent. Using Eq. 1 (Section 6.2), the distribution coefficient, D, is readily calculated for the HS RES-type MR contrast agents. For the HS MR contrast agent a value of 30.9 is obtained confirming the high degree of specificity of the HS MR contrast agent for the hepatocytes of the liver over splenic tissue. In this set of experiments, a value of 0.82 is found for AMI-25, actually indicating a preference by the RES-type MR contrast agent for the spleen. It is worth noting that the difference between this D value (0.82) and that obtained in Table II (1.23) for AMI-25 may indicate a certain intrinsic variability in biodistribution studies of mice particularly where dosages and modes of anesthesia are different. However, this slight variability in no way defeats the surprising specificity data obtained for the HS MR contrast agent, which data are obtained under substantially the same experimental conditions as the contemporaneous set of AMI-25 studies.

TABLE IV

| Changes in Tissue Relaxation Rates, Superparamagnetic HS Versus RES-Type MR Contrast Agents | | | |
|---|---|---|---|
| | Baseline | HS MR Agent | AMI-25 |
| liver | | | |
| $1/T_1$ | 3.44 | 5.14 | 4.37 |
| S.D. | 0.32 | 0.33 | 0.22 |
| n | 13 | 8 | 12 |
| spleen | | | |
| $1/T_1$ | 1.65 | 1.73 | 3.14 |
| S.D. | 0.12 | 0.1 | 0.38 |
| n | 13 | 6 | 12 |
| liver | | | |
| $1/T_2$ | 22.4 | 53.4 | 33.6 |
| S.D. | 1.7 | 6.3 | 3.0 |
| n | 13 | 8 | 12 |
| spleen | | | |
| $1/T_2$ | 16.2 | 17.3 | 29.8 |
| S.D. | 1.3 | 2.1 | 3.3 |
| n | 13 | 6 | 12 |

### 6.5. ACTION OF THE ASIALOGLYCOPROTEIN RECEPTOR IN CLEARING HS MR CONTRAST AGENTS FROM THE BLOOD

If a superparamagnetic iron oxide colloid is cleared from the blood via the asialoglycoprotein receptor, the presence of circulating asialoglycoproteins should retard its removal from the blood. To examine this hypothesis, two rats are injected with asialofetuin (100 mg/kg), two more rats with fetuin (100 mg/kg), and yet another two rats serve as a control and are not injected with glycoprotein. After allowing 5 minutes for dilution of each protein in the vascular compartment, 20 μmol/kg of the HS MR contrast agent is injected into all six animals. FIG. 4 shows the changes in blood $1/T_1$ for each group of rats. The increase in $1/T_1$ produced by the HS MR contrast agent is clearly greatly prolonged by injection of asialofetuin into rats, thus confirming the intermediacy of the asialoglycoprotein receptor in the cellular mechanism for clearing the HS MR contrast agent from the vascular compartment.

This result also demonstrates the utility of employing co-injected or pre-injected asialoglycoproteins for inhibiting the removal of the HS MR contrast agent by the capillary bed (the so-called "first pass extraction efficiency") and increasing the likelihood of obtaining useful and meaningful information about the metabolic state of the liver hepatocytes.

6.6. MR IMAGES PRODUCED BY ADMINISTRATION OF THE HS MR CONTRAST AGENTS OF THE PRESENT INVENTION

Finally the HS MR contrast agent is examined for its efficacy as an MR contrast agent. The top right image of FIG. 5 shows a coronal image of an uninjected rat, while the top left image is taken about 15 minutes after the injection of 20 μmol Fe/kg of the HS MR contrast agent. The plane of the coronal images is chosen to permit visualization of both the liver and the spleen. After injection of the HS MR contrast agent the liver darkens (upper left) dramatically, but there is a lack of darkening of the spleen (lower right, as defined by the arrow). The bottom image shows that a darkened spleen is obtained when the same rat is injected with 100 μmol Fe/kg of the RES-type MR contrast agent, AMI-25. By both tissue relaxation rate data (Table IV), or MR imaging (FIG. 5), the HS MR contrast agent produces dramatic effects on liver but not splenic relaxation rates.

6.7. TOXICOLOGY

It has been found that the injection of 1800 μmol Fe/kg fails to produce any adverse effects in rats over the standard observation period of 48 hours. This dose corresponds to about 90 times the imaging dose used in FIG. 5 (20 μmol/kg). The safety factor for the HS MR agent, i.e., $LD_{50}$/imaging dose, is, therefore, at least 90.

## Claims

**1.** A composition comprising (1) colloidal biodegradable superparamagnetic metal oxide particles associated with (2) a polysaccharide, wherein such polysaccharide is capable of being recognized and internalized, thereby permitting said metal oxide particles to be internalized, by a targeted organ or tissue by receptor mediated endocytosis.

**2.** The composition of claim 1 in which said metal oxide particles are directly associated with the polysaccharide without the use of a macromolecular species conjugate.

**3.** The composition of claim 1-2 in which the metal of said metal oxide particles is iron.

**4.** The composition of claim 1-3 in which the polysaccharide contains a terminal galactose moiety.

**5.** The composition of claim 1-2 in which the metal is iron, the polysaccharide contains a terminal galactose moiety, and the galactose/iron molar ratio is between 0.0001 and 1.0.

**6.** The composition according to claim 1-5 in which said polysaccharide is arabinogalactan.

**7.** The composition according to claim 1-6 in which said polysaccharide is the first macromolecular species of a macromolecular species conjugate which comprises the polysaccharide and a second macromolecular conjugate which is associated with the metal oxide particles and conjugated to the polysaccharide.

**8.** The composition according to claim 7 in which the second macromolecular species of the macromolecular species conjugate is a carbohydrate.

**9.** The composition according to claim 8 in which said carbohydrate is dextran.

**10.** The composition according to claim 1-6 in which the second macromolecular species of the macromolecular species conjugate is an organosilane.

11. The composition according to claim 10 in which said organosilane is selected from the group consisting of 3-aminopropyltrimethoxysilane, p-aminophenyltrimethoxysilane, N-2-aminoethyl-3-aminopropyltrimethoxysilane, n-dodecyltriethoxysilane, and n-hexyltrimethoxysilane.

12. The composition of claim 4 which is further characterized as comprising biodegradable superparamagnetic particles which measure 3000 angstroms (300 nm) or less and as having a distribution coefficient, D, favoring the hepatocytes of the liver over a reference organ or tissue of at least about 3.

13. The composition of claim 4 which is further characterized as comprising biodegradable superparamagnetic particles which measure 3000 angstroms (300 nm) or less and as having a distribution coefficient, D, favoring the hepatocytes of the liver over a reference organ or tissue of at least about 20.

14. The composition of claim 1-13 in which said particles have an overall mean diameter of about 3000 angstroms (300 nm) or less, as measured by light scattering.

15. The composition of claim 1-13 in which said particles have an overall mean diameter of about 1000 angstroms (100 nm) or less, as measured by light scattering.

16. A method for obtaining an enhanced MR image of an organ or tissue of an animal or human subject which comprises recording the MR image generated after the administration to a subject of an effect amount of the composition defined in claims 1-15 present in a physiologically acceptable medium.

**Patentansprüche**

1. Eine Verbindung bestehend aus (1) kolloidalen biologisch abbaubaren superparamagnetischen Metalloxidpartikeln in Kombination mit (2) einem Polysaccharid, dadurch gekennzeichnet, daß dieses Polysaccharid von einem Zielorgan oder Zielgewebe durch Rezeptorvermittelte Endozytose erkannt und aufgenommen werden kann und somit die Aufnahme der besagten Metalloxidpartikel ermöglicht.

2. Die Verbindung gemäß Anspruch 1, dadurch gekennzeichnet, daß besagte Metalloxidpartikel direkt ohne die Hilfe eines Konjugats der makromolekularen Art mit dem Polysaccharid verbunden sind.

3. Die Verbindung gemäß den Ansprüchen 1 und 2, dadurch gekennzeichnet, daß es sich bei dem Metall der besagten Metalloxidpartikel um Eisen handelt.

4. Die Verbindung gemäß den Ansprüchen 1 bis 3, dadurch gekennzeichnet, daß das Polysaccharid einen terminalen Galaktose-Bestandteil aufweist.

5. Die Verbindung gemäß den Ansprüchen 1 bis 2, dadurch gekennzeichnet, daß es sich bei dem Metall um Eisen handelt, daß das Polysaccharid einen terminalen Galaktose-Bestandteil aufweist und daß das Galactose/Eisen-Molverhältnis zwischen 0,0001 und 1,0 liegt.

6. Die Verbindung gemäß den Ansprüchen 1 bis 5, dadurch gekennzeichnet, daß es sich bei dem besagten Polysaccharid um Arabinogalaktan handelt.

7. Die Verbindung gemäß den Ansprüchen 1 bis 6, dadurch gekennzeichnet, daß es sich bei dem Polysaccharid um die erste makromolekulare Art eines makromolekularartigen Konjugats handelt, das das Polysaccharid sowie ein zweites makromolekulares Konjugat umfaßt, das mit den Metalloxidpartikeln verbunden und mit dem Polysaccharid gepaart ist.

8. Die Verbindung gemäß Anspruch 7, dadurch gekennzeichnet, daß es sich bei der zweiten makromolekularen Art des makromolekularartigen Konjugats um ein Kohlehydrat handelt.

9. Die Verbindung gemäß Anspruch 8, dadurch gekennzeichnet, daß es sich bei besagtem Kohlehydrat um Dextran handelt.

10. Die Verbindung gemäß den Ansprüchen 1 bis 6, dadurch gekennzeichnet, daß es sich bei der zweiten makromolekularen Art des makromolekularartigen Konjugats um ein Organosilan handelt.

11. Die Verbindung gemäß Anspruch 10, dadurch gekennzeichnet, daß das besagte Organosilan ausgewählt wird aus der Gruppe bestehend aus 3-Aminopropyltrimethoxysilan, p-Aminophenyltrimethoxysilan, N-2-Aminoethyl-3-aminopropyltrimethoxysilan, n-Dodecyltriethoxysilan und n-Hexyltrimethoxysilan.

12. Die Verbindung gemäß Anspruch 4, desweiteren dadurch gekennzeichnet, daß sie biologisch abbaubare superparamagnetische Partikel enthält, die 3.000 Angstrom (300 nm) oder weniger messen, und daß sie einen Verteilungskoeffizienten, D, aufweist, der die Hepatozyten der Leber gegenüber einem Referenzorgan oder -gewebe mindestens um ein 3faches bevorzugt.

13. Die Verbindung gemäß Anspruch 4, desweiteren dadurch gekennzeichnet, daß sie biologisch abbaubare superparamagnetische Partikel enthält, die 3.000 Angstrom (300 nm) oder weniger messen, und daß sie einen Verteilungskoeffizienten, D, aufweist, der die Hepatozyten der Leber gegenüber einem Referenzorgan oder -gewebe mindestens um ein 20faches bevorzugt.

14. Die Verbindung gemäß den Ansprüchen 1 bis 13, dadurch gekennzeichnet, daß die besagten Partikel einen mittleren Gesamtdurchmesser von ca. 3.000 Angstrom (300 nm) oder weniger, gemessen anhand der Lichtstreuungsmethode, aufweisen.

15. Die Verbindung gemäß den Ansprüchen 1 bis 13, dadurch gekennzeichnet, daß die besagten Partikel einen mittleren Gesamtdurchmesser von ca. 1.000 Angstrom (100 nm) oder weniger, gemessen anhand der Lichtstreuungsmethode, aufweisen.

16. Ein Verfahren zur Erzielung eines besseren Magnetresonanz(MR-)Bildes von einem Organ oder Gewebe eines Menschen oder Tieres, gekennzeichnet durch den Venfahrensschritt der Aufzeichnung eines nach Verabreichung einer wirksamen Dosis der in den Ansprüchen 1 bis 15 definierten Verbindung in einem physiologisch akzeptablen Medium an den Menschen bzw. an das Tier erzeugten MR-Bildes.

**Revendications**

1. Composition comprenant (1) des particules superparamagnétiques biodégradables colloidales d'oxyde métallique associées à (2) un polysaccharide, dans laquelle ce polysaccharide est capable d'être reconnu et internalisé, ce qui permet ainsi auxdites particules d'oxyde métallique d'être internalisées, par un tissu ou un organe ciblé par endocytose induite par récepteurs.

2. Composition selon la revendication 1, dans laquelle lesdites particules d'oxyde métallique sont directement associées au polysaccharide sans l'utilisation d'un conjugué d'espèce macromoléculaire .

3. Composition selon la revendication 1 - 2, dans laquelle le métal desdites particules d'oxyde métallique est le fer.

4. Composition selon la revendication 1 - 3, dans laquelle le polysaccharide contient un fragment galactose terminal.

5. Composition selon la revendication 1 - 2, dans laquelle le métal est le fer, le polysaccharide contient un fragment galactose terminal et le rapport molaire galactose/fer est compris entre 0,0001 et 1,0.

6. Composition selon la revendication 1 - 5, dans laquelle ledit polysaccharide est l'arabinogalactane.

7. Composition selon la revendication 1 - 6, dans laquelle ledit polysaccharide est la première espèce macromoléculaire d'un conjugué d'espèces macromoléculaires qui comprend le polysaccharide et un second conjugué macromoléculaire qui est associé aux particules d'oxyde métallique et conjugué au polysaccharide.

8. Composition selon la revendication 7, dans laquelle la seconde espèce macromoléculaire du conjugué d'espèces macromoléculaires est un hydrate de carbone.

9. Composition selon la revendication 8, dans laquelle ledit hydrate de carbone est le dextranne.

10. Composition selon la revendication 1 - 6, dans laquelle la seconde espèce macromoléculaire du conjugué d'espèces macromoléculaires est un organosilane.

11. Composition selon la revendication 10, dans laquelle ledit organosilane est choisi dans le groupe comprenant le 3-aminopropyltriméthoxysilane, le p-aminophényltriméthoxysilane, le N-2-aminoéthyl-3-aminopropyltriméthoxysilane, le n-dodécyltriéthoxysilane et le n-hexyltriméthoxysilane.

12. Composition selon la revendication 4, qui se caractérise encore comme comprenant des particules superparamagnétiques biodégradables qui mesurent 3000 angströms (300 nm) ou moins et comme ayant un coefficient de distribution, D, favorisant les hépatocytes du foie sur un tissu ou un organe de référence d'au moins environ 3.

13. Composition selon la revendication 4, qui se caractérise encore comme comprenant des particules superparamagnétiques biodégradables qui mesurent 3000 angströms (300 nm) ou moins et comme ayant un coefficent de distribution, D, favorisant les hépatocytes du foie sur un tissu ou organe de référence d'au moins environ 20.

14. Composition selon la revendication 1 - 13, dans laquelle lesdites particules ont un diamètre moyen global d'environ 3000 angströms (300 nm) ou moins, tel que mesuré par dispersion de la lumière.

15. Composition selon la revendication 1 - 13, dans laquelle lesdites particules ont un diamètre moyen global d'environ 1000 angströms (100 nm) ou moins, tel que mesuré par dispersion de la lumière.

16. Procédé pour l'obtention d'une image de RM exaltée d'un organe ou tissu d'un animal ou d'un sujet humain qui comprend l'enregistrement de l'image de RM produite après l'administration à un sujet d'une quantité efficace de la composition définie dans les revendications 1 - 15 présentes dans un milieu physiologiquement acceptable.

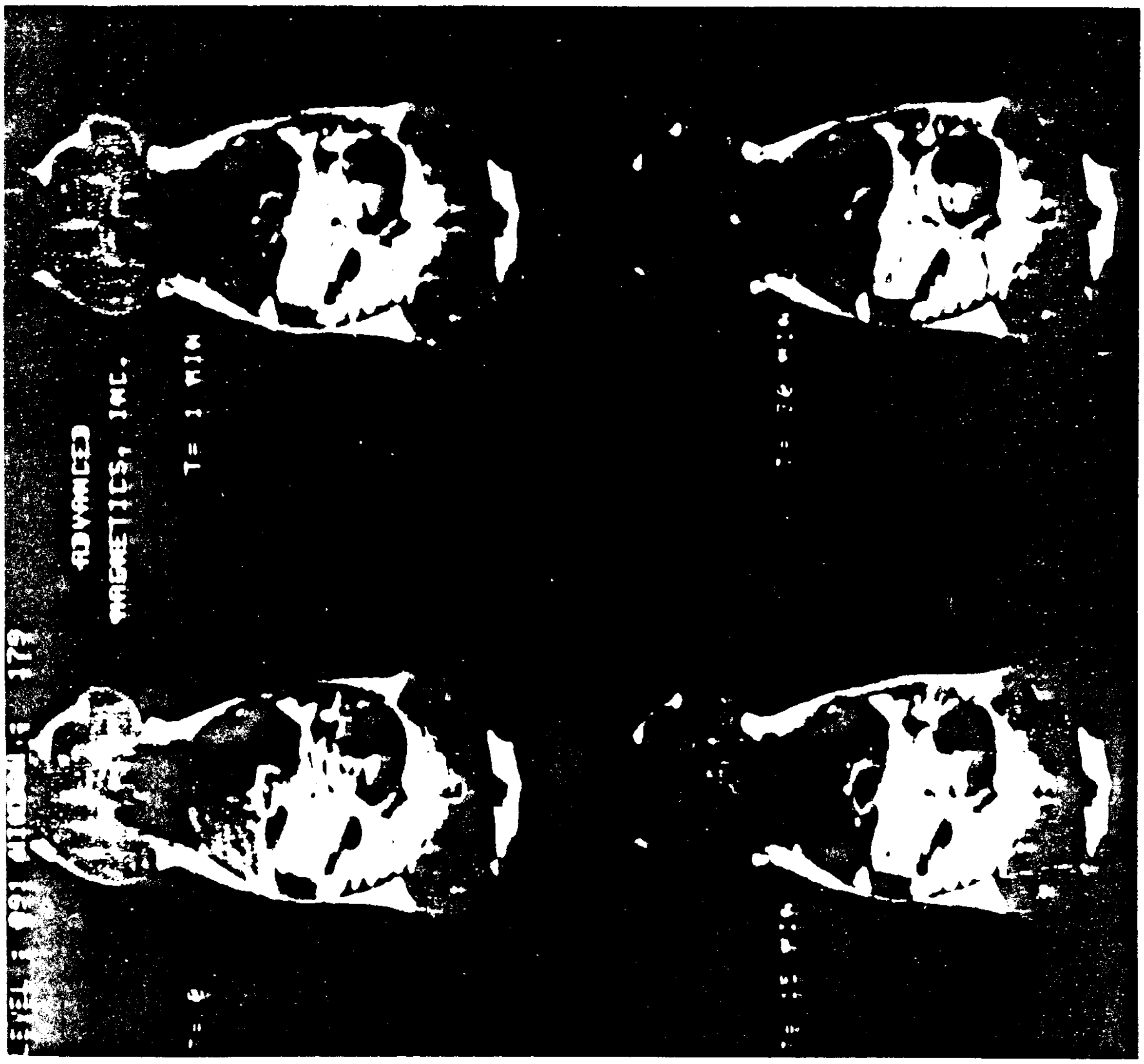


FIG. 1

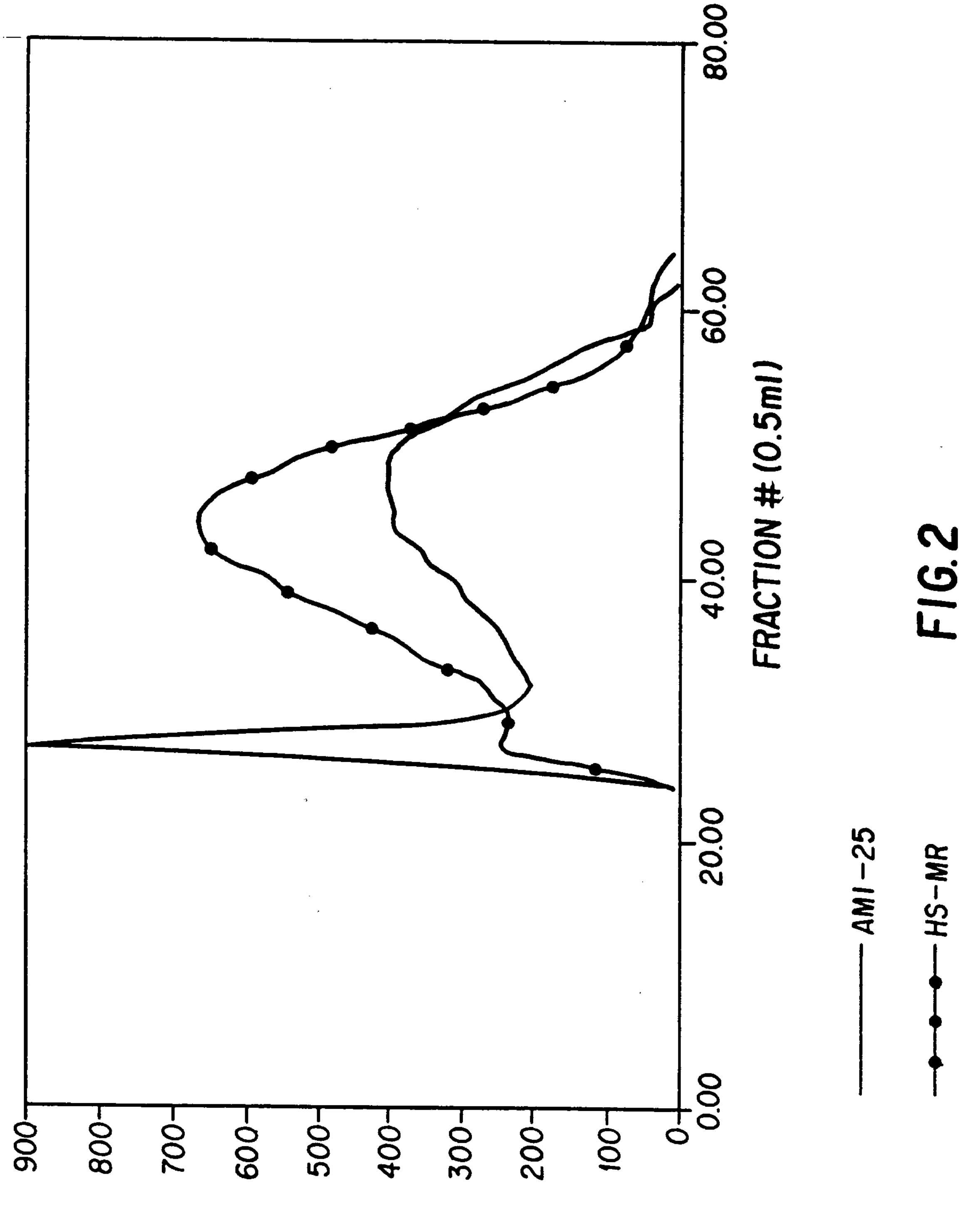
900
800
700
600
500
400
300
200
100
0
0.00
20.00
40.00
60.00
80.00
FRACTION # (0.5ml)
AMI-25
HS-MR

FIG. 2

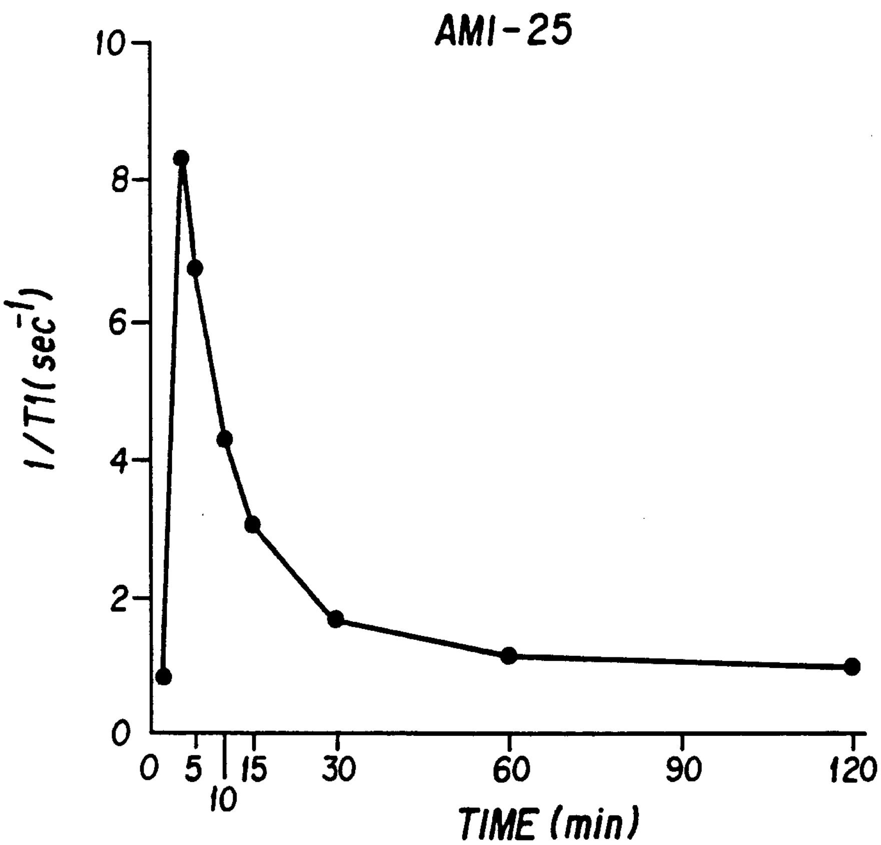
AMI-25
1/T1(sec⁻¹)
10
8
6
4
2
0
0
5
10
15
30
60
90
120
TIME (min)

FIG.3A

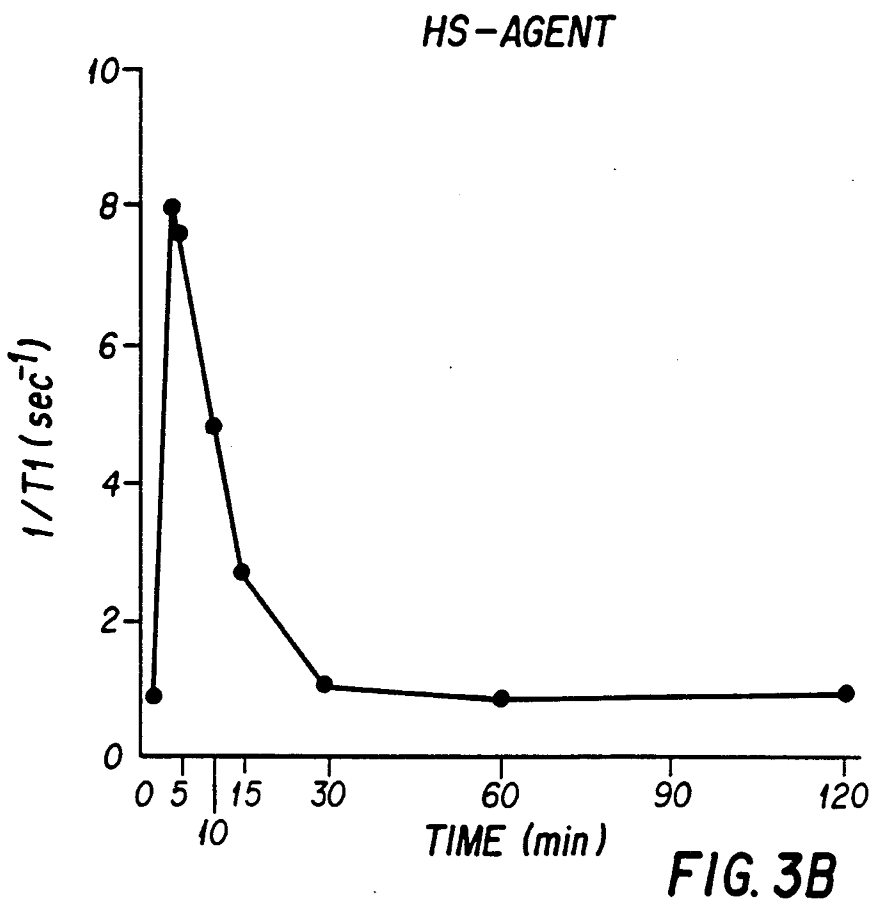
HS-AGENT
1/T1(sec⁻¹)
10
8
6
4
2
0
0
5
10
15
30
60
90
120
TIME (min)

FIG.3B

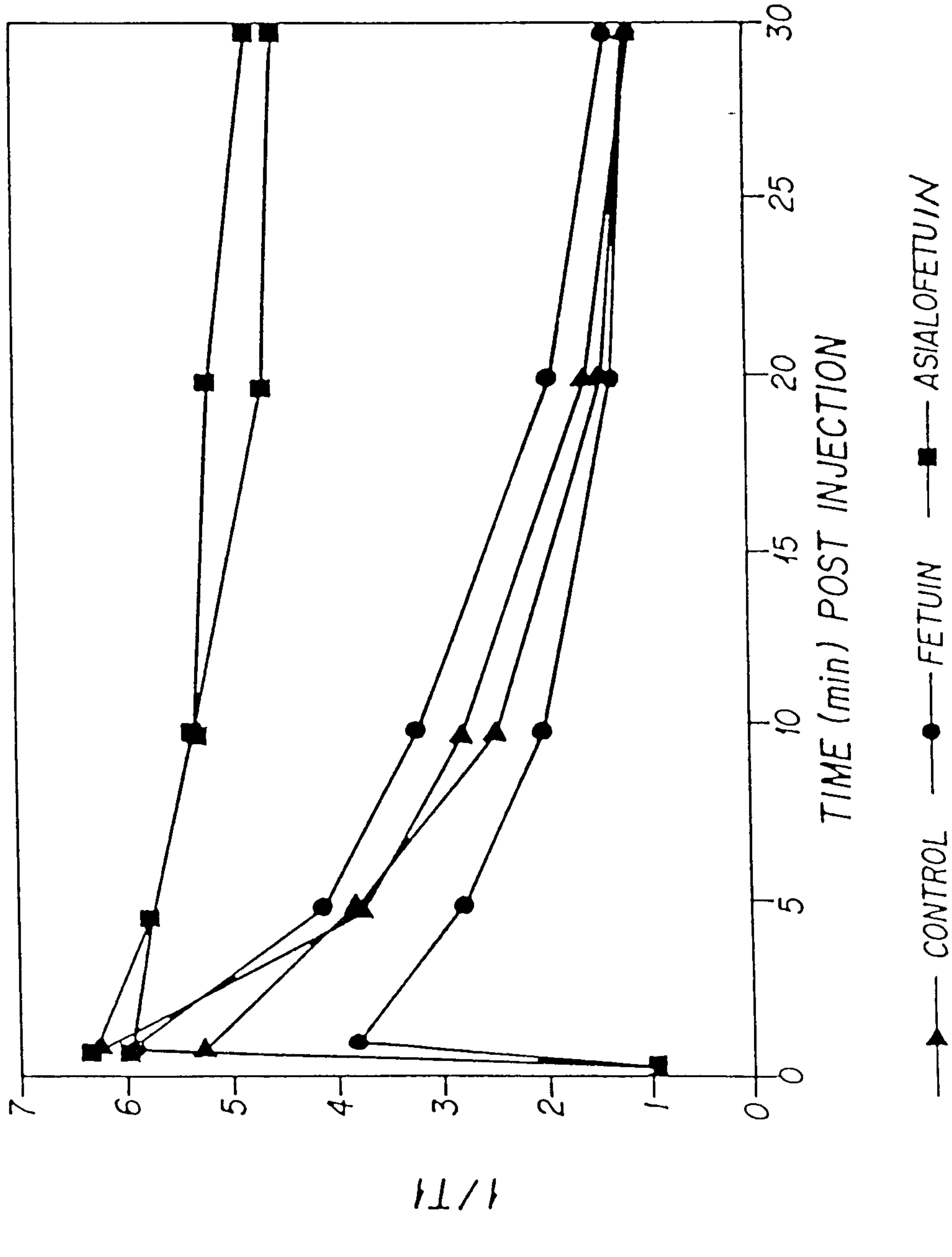
1/T1
0
1
2
3
4
5
6
7
0
5
10
15
20
25
30
TIME (min) POST INJECTION
CONTROL
FETUIN
ASIALOFETUIN

FIG. 4

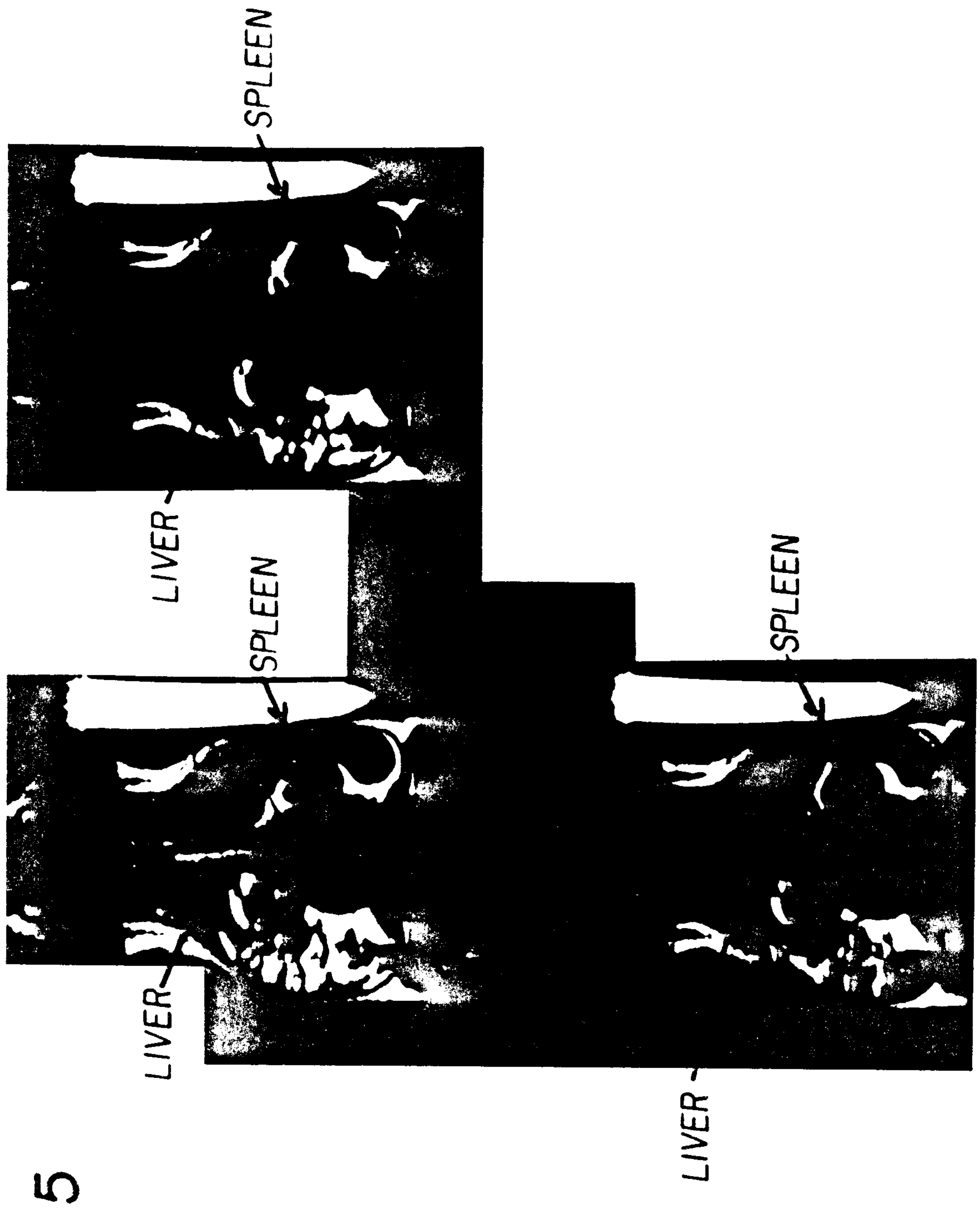


FIG. 5